# NOUVEAU FASCICULE DE BREVET EUROPEEN

(11) **EP 0 410 899 B2**
(45) Date de publication et mention de la décision concernant l'opposition: **03.02.1999**
(45) Mention de la délivrance du brevet: 28.06.1995
(21) Numéro de dépôt: 90420169.6
(22) Date de dépôt: 03.04.1990
(51) Int. Cl.: C08J 3/03, C08L 83/04, C08K 13/02, C09D 183/04

(54) **Dispersion aqueuse à base d'huiles silicones et de (co)polymère organique réticulant en un élastomère par élimination de l'eau**
Siliconöl und organisches (Co)Polymer enthaltende wässerige, nach Entfernung des Wassers zu einem Elastomer härtende Dispersion
Aqueous dispersion of silicone oils and (co)polymers which cures to an elastomer by the removal of water

(30) Priorité: 29.06.1989 FR 8909004
(43) Date de publication de la demande: 30.01.1991
(73) Titulaire: RHODIA CHIMIE, 92408 Courbevoie Cédex (FR)
(72) Inventeur: Feder, Michel, F-68720 Illfurth (FR); Jaubert, Jean-Pierre, F-95350 Saint-Brice-Sous-Forêt (FR); Pouchol, Jean-Marie, F-69006 Lyon (FR)
(74) Mandataire: Trolliet, Maurice

(56) Documents cités:
- EP-A- 0 049 026
- EP-A- 0 117 607
- EP-A- 0 117 608
- EP-A- 0 169 098
- CH-A- 618 462
- FR-A- 1 505 881
- US-A- 4 433 007

## Description

La présente invention concerne une dispersion aqueuse à base de silicone et d'un latex de polymère organique pouvant réticuler en un élastomère par élimination de l'eau.

Les dispersions aqueuses à base de silicone, pouvant réticuler en un élastomère par élimination de l'eau et comportant :
- une émulsion (A) du type huile-dans-eau d'un α,ω-(dihydroxy)polydiorganosiloxane, stabilisée par un tensio-actif anionique et/ou non-ionique,
- un agent de réticulation,
- une charge minérale non siliceuse,
- un composé catalytique de durcissement,
sont connues.

L'émulsion de base comporte l'huile silicone réactive à extrémités silanols qui est généralement polymérisée en émulsion en suivant l'enseignement des brevets US-A-2 891 920, US-A-3 294 725 et US-A-3 360 491, c'est-à-dire en utilisant un tensio-actif anionique jouant également, de préférence, le rôle de catalyseur de polymérisation.

EP-A-327 321 enseigne une dispersion aqueuse du type ci-dessus, dont l'émulsion de base est un mélange d'une macro-émulsion d'huile réactive silicone et d'une microémulsion d'huile réactive silicone de granulométrie inférieure à 0,14 µm. Une telle dispersion à le double inconvénient d'être coûteuse et de réticuler par évaporation d'eau en un élastomère présentant une adhérence sur support insuffisante et/ou des propriétés mécaniques médiocres.

Dans ce type général de dispersion aqueuse réticulant en un élastomère par élimination d'eau, la littérature enseigne la possibilité d'utiliser une grande diversité d'agents de réticulation de l'émulsion de base parmi lesquels on peut citer :
- de la silice colloïdale (US-A-3 294 725 et US-A-4 221 688),
- du silicate de soude (US-A-4 244 849),
- une silice amorphe en poudre (FR-A-2 463 163),
- une microémulsion de résine silsesquioxane (US-A-3 355 406),
- un siliconate (EP-A-266 729, EP-A-332 544),
- une résine silicone réactive de faible masse moléculaire présentant des groupes alcoxy ou acyloxy (US-A-4 554 187),
- une résine silicone de haute masse moléculaire insoluble dans le toluène (EP-A-304 719),
- un polyalcoxysilane, un polysilicate, un polyacyloxysilane ou un polycétiminoxysilane (US-A-3 294 725, US-A-4 584 341; US-A-4 618 642, US-A-4 608 412),
- un polyamino (ou amido)silane (EP-A-387 157),
- un polyalcénoxysilane (EP-A-364 375),
- une résine silicone hydroxylée présentant par molécule au moins deux motifs siloxy choisis parmi ceux de formules : R₃SiO_{0,5} (M), R₂SiO (D), RSiO_{1,5} (T) et SiO₂ (Q) (EP-A-359 676).

Ces dispersions aqueuses sont généralement catalysées par un composé catalytique de durcissement qui est, de préférence, un sel d'étain, et qui peut être également l'association d'un sel d'étain et d'acide borique (US-A-4 863 985).

Ces dispersions aqueuses présentent toutefois de nombreux inconvénients parmi lesquels on peut citer :
- une stabilité au stockage insuffisante,
- une adhérence médiocre sur de nombreux supports en particulier ceux utilisés dans l'industrie du bâtiment (verre, béton, métaux, acier, aluminium, revêtements épais en matière plastique telle que le PVC, pierres calcaires),
- les peintures préparées à partir de ces dispersions aqueuses présentent un pouvoir liant et une résistance à l'abrasion, en particulier à l'abrasion humide, qui peuvent être insuffisants,
- les élastomères obtenus à partir de ces dispersions aqueuses par évaporation de l'eau présentent un retrait très important, associé à un module élastique (ME) et une résistance à la déchirure (R/D) trop faibles pour certaines applications,
- une trop forte viscosité des émulsions silicones à forts extraits secs nécessaires à la formulation de dispersions aqueuses chargées à faible extrait volumique.

Par ailleurs, les dispersions aqueuses latex de (co)polymère organique par exemple de (méth)acrylate d'alkyle et d'esters vinyliques d'acides monocarboxyliques, sont utilisés depuis longtemps comme peintures en revêtements minces ou en revêtements épais, en particulier dans l'industrie du bâtiment pour l'hydrofugation des façades, des toitures (roofing).

Ces dispersions aqueuses présentent généralement les inconvénients suivants :
- le revêtement formé présente une perméabilité aux gaz et en particulier, à la vapeur d'eau, insuffisante,
- la viscosité de la dispersion aqueuse est trop faible et son caractère filmogène peut être insuffisant,
- les revêtements obtenus sont trop sensibles à l'eau et insuffisamment hydrofuges,
- les revêtements obtenus sont sensibles au rayonnement actinique et en particulier aux U.V.

Des documents brevets enseignent déjà l'association de silicone et d'un latex organique :
- FR-A-2 526 033, US-A-4 012 355 et US-A-2 738 910 enseignent l'association de siliconate et d'un latex d'un polymère organique,
- selon DE-A-2 355 813, le siliconate peut être remplacé par une émulsion de résine silicone et selon CH-A-618 462 à la fois du siliconate et une résine silicone sont associés à un copolymérisat d'acide acrylique.

Aucun de ces documents n'enseignent l'utilisation en outre d'une émulsion d'huile silicone à extrémités silanol, de charge non siliceuse et de catalyseurs de durcissement.

EP-A-246 537 décrit l'obtention sous forme de latex d'un matériau élastomère stratifié composé des trois éléments suivants :
- un noyau constitué de caoutchouc silicone réticulé,
- une première enveloppe constituée de caoutchouc acrylique réticulé, et
- une deuxième enveloppe obtenue par (co)polymérisation radicalaire de monomères capables de former une résine.

Un but de la présente invention est de proposer une dispersion aqueuse de silicone et d'un latex de polymère organique qui présente les avantages cumulés des dispersions aqueuses de silicone et de dispersions aqueuses de polymère organique avec, pour certaines propriétés, l'apparition d'un effet de synergie bénéfique.

Un autre but de la présente invention est de proposer une dispersion du type ci-dessus qui ne présente pas les inconvénients inhérents aux dispersions de silicone seules, ou de polymères organiques seuls, ces inconvénients pouvant subsister sous une forme atténuée, tolérable dans la plupart des utilisations de telles dispersions.

Un autre but de la présente invention est de proposer une dispersion du type ci-dessus présentant éventuellement après réticulation, à la fois les propriétés suivantes :
- une stabilité au stockage pendant au moins six mois, de préférence au moins un an,
- une viscosité du produit non chargé et du produit en cartouche convenable même pour des dispersions aqueuses présentant un extrait sec élevé supérieur à 75 %, ces dispersions conduisant à des élastomères à module peu élevé,
- une adhérence satisfaisante sur les supports les plus divers, pierre, béton, mortier, métaux, acier, aluminium, fibrociment, émaux, céramiques, matière plastique telle que le PVC,
- une viscosité convenable leur conférant une extrudabilité de la cartouche d'emballage et un caractère filmogène convenable,
- une résistance à l'abrasion, en particulier à l'abrasion humide, à l'humidité atmosphérique, aux rayonnements actiniques (lumière visible, U.V.) convenable,
- une bonne perméabilité aux gaz et à la vapeur d'eau,
- des propriétés mécaniques satisfaisantes, en particulier pour le module élastique et la résistance à la déchirure,
- un caractère hydrofuge satisfaisant.

Ces buts, et d'autres sont atteints par la présente invention qui concerne en effet une dispersion aqueuse à base de silicone, réticulant en un élastomère par élimination de l'eau dans les conditions ambiantes, caractérisée en ce qu'elle comporte en poids :
(A) 100 parties d'une émulsion, du type huile-dans-eau d'un α,ω-(dihydroxy)-polydiorganosiloxane de viscosité dynamique d'au moins 50 000 mPa.s à 25°C, émulsion stabilisée par au moins un agent tensio-actif choisi parmi les agents tensio-actifs anioniques et non-ioniques et leurs mélanges et ayant une granulométrie comprise entre 0,15 µm et 100 µm,
(B) 2 à 80 parties, de préférence 3 à 40, d'une dispersion aqueuse d'un (co)polymère organique présentant une taille de particules comprise entre 0,01 et 0,15 µm, de préférence entre 0,01 et 0,1 µm et un extrait sec compris entre 20 et 70 % en poids, le (co)polymère organique étant choisi parmi les copolymères styrène/acrylate d'alkyle dont le rapport pondéral styrène/acrylate varie entre 30/70 et 70/30, le polyacétate de vinyle homopolymère et le polystyrène homopolymère,
(C) une quantité efficace d'au moins un agent de réticulation choisi parmi :
   - 1 à 100 parties d'une microémulsion de résine silsesquioxane selon l'enseignement combiné de US-A-3 355 406 et US-A-3 433 780,
   - 5 à 100 parties d'une résine silicone réactive de faible masse moléculaire présentant des groupes alkoxy et acyloxy,
   - 5 à 100 parties d'une résine silicone de haute masse moléculaire insoluble dans le toluène,
   - 5 à 100 parties d'une résine silicone hydroxylée présentant par molécule, au moins deux motifs différents choisis parmi ceux de formules : R₃SiO_{0,5} (M), R₂SiO (D), RSiO_{1,5} (T) et SiO₂ (Q), R étant principalement un radical alkyle en C₁ - C₆, vinyle et trifluoro-3,3,3 propyle et une teneur en poids en groupe hydroxyle comprise entre 0,1 et 10 %,
(D) 5 à 200 parties, de préférence 50 à 150, d'une charge minérale non siliceuse,
(E) éventuellement 0,01 à 3 parties en poids d'un composé métallique catalytique de durcissement,
ladite dispersion ayant une teneur en extrait sec supérieure à 60 % en poids et une viscosité à 25°C inférieure à 50 000 mPa.s mesurée à un gradient de vitesse de 1.s⁻¹ avant l'incorporation des charges (D).

La dispersion aqueuse finale est préparée par simple mélange intime de tous ses constituants conduisant à une dispersion homogène, stable au stockage à l'abri de l'air.

### - L'EMULSION (A) :

Les α,ω-(dihydroxy)-polydiorganosiloxanes doivent avoir une viscosité à 25°C, d'au moins 50 000 mPa.s.

C'est en effet pour des viscosités supérieures à 50 000 mPa.s que l'on obtient un élastomère présentant un ensemble de propriétés mécaniques convenables, en particulier concernant la dureté Shore A et l'allongement.

En outre plus la viscosité est élevée et plus les propriétés mécaniques se conservent lors du vieillissement de l'élastomère.

Les viscosités préférées pour la présente invention sont comprises entre 50 000 et 1 500 000 mPa.s à 25°C.

Les radicaux organiques des α,ω-(dihydroxy)-polydiorganosiloxanes sont des radicaux hydrocarbonés monovalents contenant jusqu'à 6 atomes de carbone, éventuellement substitué par des groupes cyano ou fluoro. Les substituants généralement utilisés du fait de leur disponibilité dans les produits industriels sont les radicaux méthyle, éthyle, propyle, phényle, vinyle et 3,3,3-trifluoropropyle. En général au moins 80 % en nombre de ces radicaux sont des radicaux méthyle.

Dans le cadre de la présente invention on préfère plus spécialement utiliser les α,ω-(dihydroxy)-polydiorganosiloxanes préparés par le procédé de polymérisation anionique décrit dans les brevets américains précités : US-A-2 891 920 et surtout US-A-3 294 725 (cités comme référence). Le polymère obtenu est stabilisé anioniquement par un agent tensio-actif qui, conformément à l'enseignement de US-A-3 294 725 est de préférence le sel d'un métal alcalin d'un acide hydrocarboné aromatique sulfonique, l'acide libre jouant également le rôle de catalyseur de polymérisation.

Le catalyseur et l'agent tensio-actif préférés sont l'acide dodécylbenzènesulfonique et ses sels de métaux alcalins, en particulier son sel de sodium. On peut ajouter éventuellement d'autres agents tensio-actifs anioniques ou non-ioniques. Toutefois cet ajout n'est pas nécessaire car, conformément à l'enseignement de US-A-3 294 725, la quantité d'agent tensio-acitf anionique résultant de la neutralisation de l'acide sulfonique est suffisante pour stabiliser l'émulsion de polymère. Cette quantité est généralement inférieure à 3 %, de préférence 1,5 % du poids de l'émulsion.

Ce procédé de polymérisation en émulsion est particulièrement intéressant car il permet d'obtenir directement l'émulsion (A). Par ailleurs ce procédé permet de pouvoir obtenir sans difficulté des émulsions (A) de α,ω-(dihydroxy)-polydiorganosiloxane de très haute viscosité.

Pour préparer l'émulsion (A) on peut également partir d'α,ω-(dihydroxy)-polydiorganosiloxane déjà polymérisé, puis le mettre en émulsion aqueuse en stabilisant les émulsions par un agent tensio-actif anionique et/ou non-ionique suivant un procédé bien connu de l'homme de métier et décrit en détail dans la littérature (voir par exemple les brevets FR-A-2 064 563, FR-A-2 094 322, FR-A-2 114 230 et EP-A-169 098).

Selon ce procédé, on mélange par simple agitation les polymères α,ω-(dihydroxy)-polydiorganosiloxane avec l'agent tensio-actif anionique ou non-ionique, ce dernier pouvant être en solution aqueuse, à ajouter ensuite si nécessaire de l'eau et à transformer l'ensemble en une émulsion fine et homogène par passage dans un broyeur classique à colloïdes.

Par la suite le broyat obtenu est dilué par une quantité d'eau appropriée et on obtient ainsi une émulsion (A) stabilisée par un agent tensio-actif anionique ou non-ionique stable au stockage.

La quantité d'agent tensio-actif anionique et non-ionique utilisable est celle utilisée couramment pour la mise en oeuvre du procédé de mise en émulsion, en particulier ceux décrits dans les brevets précités et dans le brevet US-A-2 891 920.

Dans le cadre de la présente invention les agents tensio-actifs anioniques préférés sont le sel d'un métal alcalin d'un acide hydrocarboné aromatique sulfonique et les agents tensio-actifs non-ioniques préférés sont les alkylphénols polyoxyéthylénés. Ces agents tensio-actifs non-ioniques sont bien entendu les mêmes que ceux que l'on peut ajouter éventuellement aux émulsions (A) obtenues par polymérisation en émulsion comme indiqué plus haut.

L'émulsion (A) préparée par polymérisation en émulsion ou par mise en émulsion du polymère silicone se présente sous la forme d'une émulsion huile dans l'eau ayant une granulométrie comprise entre 0,15 µm et 100 µm et a, de préférence, une teneur en extrait sec d'au moins 40 % en poids.

### - LA DISPERSION (B) :

C'est un "latex" formé d'une dispersion aqueuse de particules de polymères issus de procédés classiques de (co)polymérisation en émulsion de monomères organiques polymérisables. Les (co)polymères organiques constitutifs des latex sont choisis, comme indiqué ci-avant, parmi : les copolymères styrène/acrylate d'alkyle dont le rapport pondéral styrène/acrylate varie entre 30/70 et 70/30, le polyacétate de vinyle homopolymère et le polystyrène homopolymère.

En ce qui concerne les monomères acrylate d'alkyle, ils sont choisis parmi les espèces de ce type dont la partie alkyle comporte de préférence de 1 à 18 atomes de carbone, en particulier l'acrylate de méthyle, l'acrylate d'éthyle, l'acrylate de propyle, l'acrylate de n-butyle, l'acrylate d'isobutyle, l'acrylate d'amyle, l'acrylate de lauryle, l'acrylate d'isoamyle, l'acrylate de (2 éthyl-2 hexyle), l'acrylate d'octyle, le chloroacrylate de butyle, le chloroacrylate de méthyle, le chloroacrylate d'éthyle, le chloroacrylate d'isopropyle, le chloroacrylate de cyclohexyle.

La stabilisation des particules de polymère au sein des dispersions aqueuses est assurée au besoin par des émulsifiants (tensio-actifs), de préférence non-ioniques tels que les alcools gras polyéthoxylés, les alkylphénolpolyéthoxylés et les acides gras polyéthoxylés, ou anioniques tels que les alkylsulfates, les alkylsulfonates, les alkylarylsulfonates (en particulier le dodécylbenzènesulfonate et les dialkylsulfosuccinates).

Les dispersions aqueuses de polymères organiques ci-dessus sont décrites en détail dans la littérature. Elles présentent dans le cadre de la présente invention un extrait sec compris généralement entre 20 et 70 % en poids et une taille de particules comprise entre 0,01 et 0,15 µm.

Ces dispersions aqueuses (B) permettent précisément d'augmenter la teneur en extrait sec des dispersions aqueuses en cartouche tout en conservant une viscosité convenable. Ces dispersions aqueuses finales conduisent, après évaporation de l'eau, à des élastomères à bas module et à plus faible retrait volumique.

Pour aboutir à un tel élastomère, la dispersion aqueuse selon l'invention résulte du mélange de l'émulsion (A) et de la dispersion (B), et présente comme indiqué ci-avant :
- une granulométrie de particules "bipopulée", c'est-à-dire une première population de particules provenant essentiellement de la dispersion (B) dont la granulométrie est comprise entre 0,01 µm et 0,15 µm, de préférence entre 0,01 µm et 0,10 µm et une deuxième population de particules provenant essentiellement de l'émulsion (A) et présentant une granulométrie comprise entre 0,15 µm et 100 µm, généralement entre 0,20 µm et 5 µm,
- un extrait sec supérieur à 60 % en poids, de préférence supérieur à 70 % en poids, et
- une viscosité à 25°C inférieure à 50 000 mPa.s mesurée à un gradient de vitesse de 1.s⁻¹, avant l'incorporation des charges (D), de façon à obtenir une dispersion finale qui puisse être extrudée facilement hors de la cartouche de stockage.

Pour préparer de telles dispersions bipopulées, il est recommandé selon l'invention d'effectuer la polymérisation en émulsion de l'émulsion (A), en présence de la dispersion (B).

Dans le cadre de la présente invention on peut utiliser des latex (dispersion B) filmogènes ou non-filmogènes.

Par latex filmogène on entend un latex dont les particules de polymère coalescent pour former une pellicule à la température d'application de la dispersion finale. Cette température peut varier entre 5 et 45°C. Elle se situe généralement autour de 20°C. Réciproquement les latex non-filmogènes sont ceux dont les particules de polymère subsistent en tant qu'entités discrètes dans le matériau final et ne coalescent pas à la température d'application de la dispersion finale, c'est-à-dire lors de l'évaporation de l'eau et sa transformation en élastomère.

L'homme du métier sait préparer des latex filmogènes ou non-filmogènes (voir en particulier US-A-3 819 557 cité comme référence pour les latex non-filmogènes) en choisissant le ou les monomères, en adaptant le procédé de polymérisation en émulsion et en ajoutant éventuellement un solvant. Pour obtenir un latex non-filmogène il est généralement suffisant que le (co)polymère organique présente une température de transition vitreuse supérieure à la température de formation de l'élastomère à partir de la dispersion finale, c'est-à-dire supérieure à 45°C. L'utilisation d'un latex filmogène est cependant préférée car on aboutit à un élastomère ayant des propriétés de souplesse améliorées.

### L'AGENT DE RETICULATION (C) :

Cet agent de réticulation permet, lors de la réticulation de la dispersion aqueuse, de bâtir le réseau élastomère par des réactions de polycondensation entre les groupes réactifs de l'agent de réticulation et les extrémités silanol de l'huile silicone de l'émulsion (A).

Parmi les résines silicones hydroxylées qui peuvent être utilisées, ces dernières étant introduites telles quelles ou sous la forme d'émulsions aqueuses, on peut citer les résines MQ, MDQ, TD et MTD.

### LA CHARGE MINERALE NON SILICEUSE (D) :

Un autre constituant de la dispersion selon l'invention est l'addition de 5 à 200, de préférence de 50 à 150 parties d'une charge minérale semi-renforçante ou de bourrage (D).

Les charges (D) ont une granulométrie généralement comprise entre 0,001 et 300 µm et une surface BET inférieure à 100 m²/g.

Des exemples de charges (D) utilisables seules ou en mélange sont le noir de carbone, le dioxyde de titane, l'oxyde d'aluminium, l'alumine hydratée, la vermiculite expansée, la vermiculite non expansée, le borax hydraté, le carbonate de calcium, l'oxyde de zinc, le mica, le talc, l'oxyde de fer, le sulfate de baryum et la chaux éteinte.

Ces charges (D) sont introduites dans l'émulsion sous forme de poudre sèche par exemple par simple mélange.

Selon une variante de l'invention, on a découvert que si la charge (D) n'est sensiblement constituée que d'une charge choisie parmi l'alumine hydratée, la vermiculite expansée, la vermiculite non expansée, le borax hydraté selon une teneur de 5 à 200, de préférence de 50 à 150 parties pour 100 parties d'émulsion (A) on obtient un élastomère ayant une résistance à la flamme particulièrement élevée qui ne peut pas être obtenue avec les autres catégories de charge (D) précitées, en particulier avec l'oxyde d'aluminium ou alumine non hydratée. On peut également incorporer des fibres céramiques ou d'aramide selon l'enseignement de EP-A-212 827.

Selon une variante, on peut incorporer en outre, pour 100 parties d'émulsion (A) un additif silicié (F) consistant dans une charge siliceuse renforçante ou semi-renforçante (1 à 100 parties).

Ces charges siliceuses sont choisies parmi les poudres de silice de combustion et de précipitation ou leur mélange. La silice de combustion est préférée. On peut toutefois utiliser également des charges siliceuses semi-renforçantes telles que des terres de diatomées, du quartz broyé.

Pour 100 parties d'émulsion (A), la somme des parties de (D) + (F) doit être inférieure à 350 parties.

Les poudres de silice de combustion et de précipitation sont bien connues, elles sont utilisées en particulier comme charges dans les compositions élastomère de silicone, vulcanisables à chaud en caoutchouc de silicone. Ces poudres présentent une taille moyenne de particule généralement inférieure à 0,1 µm et une surface spécifique BET supérieure à 50 m²/g, de préférence comprise entre 150 et 350 m²/g.

L'incorporation dans l'émulsion (A) de cet additif silicié (F), par tout moyen convenable, en particulier par agitation, augmente considérablement la viscosité de l'émulsion (A) qui présente alors un caractère pâteux.

On a en effet trouvé, conformément à la présente invention que l'addition de cet additif silicié (F), est suffisante pour conférer à l'émulsion un caractère "thixotrope" plus ou moins marqué. L'émulsion, extraite par exemple d'une cartouche de stockage, adhère sans s'écouler sur un substrat même vertical et durcit en élastomère par évaporation de l'eau à température ambiante. On peut également obtenir une émulsion non coulante en utilisant comme charge (D) du carbonate de calcium de précipitation dont le diamètre particulaire moyen est inférieur à 0,1 µm.

### LE COMPOSE METALLIQUE CATALYTIQUE DE DURCISSEMENT (E):

Le composé (E) est facultatif, mais dans le cadre de la présente invention, il est recommandé d'utiliser (E).

Les composés catalytiques métalliques de durcissement (E) sont essentiellement les sels d'acides carboxyliques et les halogénures de métaux choisis parmi le plomb, le zinc, le zirconium, le titane, le fer, l'étain, le baryum, le calcium et le manganèse.

Le constituant (E) est de préférence un composé catalytique à l'étain, généralement un sel d'organoétain, introduit de préférence sous forme d'une émulsion aqueuse. Les sels d'organoétain utilisables sont décrits, en particulier dans l'ouvrage de NOLL, Chemistry and Technology of Silicones Academic Press (1968), page 337.

On peut également utiliser le produit de réaction d'un silicate d'alkyle ou d'un alkyltrialkoxysilane sur le diacétate de dibutylétain comme décrit dans le brevet belge BE-A-842 305.

Les sels d'étain préférés sont les bischélates d'étain (EP-A-147 323 et EP-A-235 049), les dicarboxylates de diorganoétain et en particulier les diversatates de dibutyl- ou de dioctylétain (brevet britannique GB-A-1 289 900), le diacétate de dibutylou de dioctylétain, le dilaurate de dibutyl- ou dioctylétain. On utilise de 0,01 à 3, de préférence de 0,05 à 2 parties de sel d'organoétain pour 100 parties de (A).

Les dispersions aqueuses selon l'invention peuvent comporter en outre des additifs usuels tels que notamment des antifongiques, des antimousses, des antigels tels que l'éthylène glycol et le propylène glycol, et des agents thixotropants tels que la carboxyméthylcellulose, la gomme xanthane et l'alcool polyvinylique, des agents dispersants (phosphates), des plastifiants (huiles silicones non réactives, plastifiants organiques tels que les alkylbenzènes de poids moléculaire supérieur à 200).

Pour préparer les dispersions aqueuses selon l'invention, il est recommandé d'ajouter sous agitation à température ambiante à l'émulsion (A), en premier lieu la dispersion (B) puis l'agent de réticulation (C) éventuellement sous la forme d'une dispersion ou d'une émulsion aqueuse, ensuite le catalyseur métallique de durcissement (E) et enfin la charge minérale non siliceuse (D) et éventuellement l'additif silicié (F).

Le pH de la dispersion aqueuse peut être acide, neutre ou basique. Il est toutefois recommandé de régler le pH de la dispersion à une valeur comprise entre 8 et 13 au moyen d'une base minérale ou organique forte (triéthanolamine, soude, potasse).

La dispersion finale obtenue est homogénéisée puis dégazée et est ensuite conditionnée en emballage étanche à l'oxygène de l'air et à la vapeur d'eau.

Les constituants (A), (B), (C), (D) et (E) et éventuellement (F) sont mélangés en des quantités telles que l'émulsion finale présente une teneur en extrait sec supérieure à 60 % en poids mais généralement inférieure à 90 %. La zone de pH préférée est comprise entre 8 et 13.

Les dispersions selon l'invention peuvent être utilisées comme peinture réticulable en couche mince. Elles présentent alors, de préférence, un extrait sec au plus égal à 70 %.

Pour déterminer la teneur en extrait sec on place 2 g de dispersion dans une coupelle de pesage en aluminium et on la chauffe une heure à 150°C dans un four à circulation d'air. Après refroidissement on pèse à nouveau la coupelle et on détermine le pourcentage de matière restante sur les 2 g initiaux qui représente la teneur en extrait sec.

Selon une variante préférée, la dispersion selon l'invention après sa préparation subit une étape de maturation, à température ambiante, de quelques heures à quelques jours.

Cette étape de maturation consiste simplement à laisser reposer la dispersion à l'abri de l'oxygène de l'air avant son utilisation.

Les dispersions selon l'invention peuvent être utilisées pour la réalisation de joints élastomères silicone, en particulier pour le bâtiment et comme revêtements hydrofuges de surfaces de bâtiment en contact avec les intempéries, à la dose par exemple de 20 à 100 g de dispersion par m² de surface à revêtir.

Ces dispersions sont également utilisables pour l'enrobage de divers principes actifs pharmaceutiques ou phytosanitaires formulés sous une forme solide (pastilles, tablettes, pilules, etc ...), pour l'enduction de bouchons de liège utilisés pour obturer les bouteilles de vins et de spiritueux, pour réaliser des revêtements d'objets culinaires et, de façon générale, d'objets en contact avec des aliments (par exemple des moules à pain).

Les techniques connues d'enrobage sont utilisables en particulier les techniques d'enduction au pinceau et au trempé (par immersion), les techniques de pulvérisation, les techniques d'enrobage en lit fluidisé et les techniques d'enduction par immersion.

Pour les revêtements de bouchons de liège, une technique recommandée est la technique au trempé qui consiste à immerger les bouchons dans la dispersion qui mouille la surface du bouchon, puis à évaporer l'eau.

Le revêtement obtenu représente 20 à 50 mg d'élastomère pour 100 cm² de surface de bouchon. Cette couche facilite le glissement du bouchon dans le goulot de la bouteille lors de l'embouteillage et évite le "coulage", c'est-à-dire des fuites de liquide entre le goulot et le bouchon.

Les dispersions selon l'invention sont utilisables également en cosmétologie dans le cas où leur pH est conforme à l'application particulière visée.

Ainsi les dispersions sont utilisables dans les compositions cosmétiques pour cheveux, notamment pour les permanentes en vue de créer un film élastomère poreux sur les cheveux selon l'enseignement de EP-A-240 349 et EP-A-240 350 cités comme référence.

Les dispersions sont également utilisables dans les compositions de crèmes de masques de beauté et également pour réaliser des dermocopies (reproduction du relief de la peau).

Enfin les dispersions selon l'invention sont utilisables comme compositions épilatoires en suivant l'enseignement de US-A-4 734 099 cité comme référence.

Dans tout ce qui suit ou ce qui précède, sauf mentions contraires, les pourcentages et parties sont en poids.

Les exemples suivants illustrent l'invention sans en limiter la portée.

### Exemples 1 à 4 et exemples comparatifs 5 et 6

### Préparation du broyat (A1)

On mélange sous agitation :
- 909 parties d'une huile α,ω-dihydroxypolydiméthylsiloxane de viscosité 160 mPa.s à 25°C,
- 45,4 parties de tensio-actif non ionique CEMULSOL® NP12 (nonylphénol polyéthoxylé), commercialisé par la Société RHONE-POULENC,
- 45,6 g d'eau distillée ajoutée lentement en 15 minutes.

Le mélange obtenu est passé au broyeur à colloïde et l'on obtient 1000 parties de broyat.

### Préparation de l'émulsion E1 :

A 500 parties du broyat (A1) ainsi obtenu on ajoute : 50 parties d'un latex styrène/acrylate d'alkyle, commercialisé par la Société RHONE-POULENC sous la marque RHODOPAS® DEA913 ayant les caractéristiques suivantes :
- filmogène à une température supérieure à 16°C :

| | |
|---|---|
| - extrait sec | 38 % |
| - taille des particules | 0,05 µm |

- pH = 8,5
- viscosité Brookfield (50 tours / minute) : 250 mPa.s

On ajoute ensuite 67 parties d'eau, puis, très rapidement : 79 parties d'une solution aqueuse à 20 % en poids d'acide dodécylbenzène sulfonique (DBSA). On maintient l'agitation pendant 3 heures, puis on laisse polymériser au repos pendant 21 heures supplémentaires. En fin de polymérisation on neutralise avec 15,9 parties d'une solution aqueuse à 50 % en poids de triéthanolamine pour obtenir l'émulsion E1 dont la composition finale et les caractéristiques sont rassemblées dans le tableau 1 ci-après.

### Préparation de l'émulsion E2 :

On dilue 500 parties de broyat (A1) avec 50 parties de latex RHODOPAS® DEA913, puis par 89 parties d'une solution aqueuse à 14,83 en poids de laurylsulfate de sodium et enfin avec 48,4 parties d'eau distillée. Après agitation on introduit 7,6 parties d'une solution aqueuse à 22 % en poids d'acide chlorhydrique (HCI) pour déclencher la polymérisation.

Après 24 heures au repos à 25°C, on neutralise par 13,7 g de triéthanolamine à 50 % en poids dans l'eau. La composition finale et les caractéristiques de l'émulsion E2 ainsi obtenues sont rassemblées dans le tableau 1 ci-après.

### Préparation de l'émulsion E3 :

On opère exactement de la même façon que pour préparer l'émulsion E2 sauf que :
- on ajoute 100 parties de RHODOPAS®,
- on n'ajoute pas d'eau distillée.

### Préparation de l'émulsion E4 :

Elle est obtenue par dilution de l'émulsion (E3) par une quantité adaptée d'eau distillée.

### Préparation de l'émulsion E5:

On répète le mode opératoire de préparation de l'émulsion (E1) sauf que l'on n'introduit pas de latex.

### Préparation de l'émulsion E6 :

On répète le mode opératoire de préparation de l'émulsion (E2) sauf que l'on n'introduit pas de latex.

Les compositions et les caractéristiques des émulsions E1 à E6 sont rassemblées dans le tableau 1 ci-après. La comparaison des extraits secs et des viscosités correspondantes mesurées au viscosimètre RHEOMAT®30 à 25°C a un gradient de vitesse de 1s⁻¹, illustre l'intérêt des émulsions E1 à E4 bipopulées qui permettent d'obtenir des extraits secs élevés, souvent recherchés pour les applications dans le bâtiment, tout en conservant des valeurs de viscosités faibles compatibles avec les procédés de préparation de la dispersion finale chargée.

**TABLEAU 1**

| EXEMPLE | 1 | 2 | 3 | 4 | 5 | 6 |
|---|---|---|---|---|---|---|
| EMULSION | E1 | E2 | E3 | E4 | E5 | E6 |
| Compositions : parties en poids | | | | | | |
| Huile hydroxylée silicone | 652,2 | 641,3 | 641 | 596,5 | 636,9 | 637 |
| CEMULSOL ® NP12 | 32,6 | 32,0 | 32 | 29,8 | 31,8 | 31,8 |
| Laurylsulfate de sodium | - | 18,6 | 18,7 | 17,4 | - | 18,6 |
| DBSA | 22,8 | - | - | - | 22,3 | - |
| HCl pur | - | 2,4 | 2,4 | 2,2 | - | 2,4 |
| Triéthanolamine pure | 11,4 | 9,7 | 9,7 | 9,0 | 11,2 | 9,7 |
| Eau | 253,8 | 225,5 | 155,2 | 213,8 | 297,8 | 300,5 |
| Latex RHODOPAS ® DEA913 | 27,2 | 70,5 | 141,0 | 131,0 | - | - |

| Caractéristiques | | | | | | |
|---|---|---|---|---|---|---|
| extrait sec mesuré (%) E.S.M. | 74,6 | 73,85 | 76,3 | 71,0 | 71,0 | 71,6 |
| extrait sec calculé (%) E.S.C. | 73,0 | 73,1 | 75,8 | - | - | - |
| viscosité du polymère silicone (Pa.s) | 110,0 | 150,0 | 90,0 | 90,0 | 320,0 | 220,0 |
| viscosité de l'émulsion à 1s⁻¹ (Pa.s) | 28,0 | 28,0 | 37,0 | 14,8 | 52,0 | 50,0 |
| Rapport E.S.M. viscosité émulsion | 2,66 | 2,64 | 2,06 | 4,80 | 1,36 | 1,43 |

### Exemple 7 et exemple comparatif 8 :- Formulation des dispersions aqueuses chargées D1 et D5

### Préparation de la dispersion D1

Dans un malaxeur à bras de type MEILI® de 5 litres on ajoute dans l'ordre les ingrédients suivants, en respectant à chaque introduction d'un nouvel ingrédient, une durée d'incorporation de 10 minutes :
- 134 parties d'émulsion (E1),
- 2 parties d'une solution aqueuse à 50 % en poids de potasse,
- 0,52 partie d'émulsion aqueuse à 37 % en poids de dilaurate de di n-octyl étain,
- 100 parties de CaCO₃ de précipitation, de granulométrie moyenne de 70 nanomètres,
- 7 parties d'une résine silicone hydroxylée introduite telle quelle, ayant 2,2 % en poids de groupe hydroxyle constituée de 70 % en poids de motifs CH₃SiO_{1,5} et 30 % en poids de motifs (CH₃)₂SiO. Cette résine est soluble dans le toluène, présente une masse moléculaire d'environ 1300 et un rapport molaire CH₃/Si = 1,77.

On homogénéise le mélange final obtenu encore 30 minutes sous une pression réduite de 1,33 KPa.

La composition obtenue est stockée en cartouche étanche à l'air et à température ambiante pendant environ 15 jours.

Après 15 jours de stockage, on étend à la racle la dispersion suivant une pellicule (film) de 2 mm d'épaisseur qu'on laisse sécher pendant 15 jours à température ambiante (20°C).

Sur les pellicules séchées, on mesure les propriétés mécaniques moyennes suivantes :
- la dureté Shore A (DSA) selon la norme ASTM-D-2240,
- la résistance à la rupture (R/R) selon la norme AFNOR-T-46 002 correspondant à la norme ASTMD 412, EN MPa,
- l'allongement à la rupture (A/R) en % selon la norme AFNOR-T 46 002,
- le module élastique (ME) à 100 % d'allongement selon la norme AFNOR-T 46 002, en MPa.

La composition de D1 et ses propriétés mécaniques sont rassemblées dans le tableau 2 ci-après.

### Préparation de la dispersion D5

On répète exactement le mode opératoire que celui utilisé pour D1 sauf que l'on utilise 141 parties d'émulsion (E5).

La composition de D5 et ses propriétés mécaniques sont rassemblées dans le tableau 2 ci-après.

Du tableau 2, il apparaît que l'on obtient avec D1 un élastomère ayant des propriétés mécaniques similaires à celles obtenues à partir de D5.

**TABLEAU 2**

| EXEMPLE | 7 | 8 |
|---|---|---|
| Composition (en parties en poids) | D1 | D5 |
| Emulsion D1 | 134 | - |
| Emulsion D5 | - | 141 |
| Solution KOH à 50 % | 2 | 2 |
| Emulsion Sn | 0,52 | 0,52 |
| CaCO₃ | 100 | 100 |
| Résine silicone | 7 | 7 |
| Coulabilité (test BOEING) en mm | 0 | 0 |
| Dureté SHORE A A | 30 | 28 |
| R/R (MPa) | 30 | 28 |
| A/R (%) | 678 | 908 |
| Module à 100 % (MPa) | 0,55 | 0,40 |

## Revendications

1. Dispersion aqueuse à base de silicone, réticulant en un élastomère par élimination de l'eau dans les conditions ambiantes, caractérisée en ce qu'elle comporte en poids :
(A) : 100 parties d'une émulsion, du type huile-dans-l'eau d'un α,ω-(dihydroxy)polydiorganosiloxane de viscosité dynamique d'au moins 50 000 mPa.s. à 25°C, émulsion stabilisée par au moins un tensio-actif choisi parmi les agents tensio-actifs anioniques et non-ioniques et leurs mélanges et ayant une granulométrie comprise entre 0,15 µm et 100 µm,
(B) : 2 à 80 parties, de préférence 3 à 40, d'une dispersion aqueuse (latex) d'un (co)polymère organique présentant une taille de particules comprise entre 0,01 et 0,15 µm et un extrait sec compris entre 20 et 70 % en poids, le (co)polymère organique étant choisi parmi les copolymères styrène/acrylate d'alkyle dont le rapport pondéral styrène/acrylate varie entre 30/70 et 70/30, le polyacétate de vinyle homopolymère et le polystyrène homopolymère,
(C) : une quantité efficace d'au moins un agent de réticulation choisi parmi :
. 1 à 100 parties d'une microémulsion de résine silsesquioxane,
. 5 à 100 parties d'une résine silicone réactive de faible masse moléculaire présentant des groupes alkoxy et acyloxy,
. 5 à 100 parties d'une résine silicone de haute masse moléculaire insoluble dans le toluène,
. 5 à 100 parties d'une résine silicone hydroxylée présentant par molécule, au moins 2 motifs différents choisis parmi ceux de formules : R₃SiO_{0,5} (M), R₂SiO (D), RSiO_{1,5} (T) et SiO₂ (Q), R étant principalement un radical alkyle en C₁-C₆, vinyle et trifluoro-3,3,3 propyle et une teneur en poids en groupe hydroxyle comprise entre 0,1 et 10 %,
(D) : 5 à 200 parties, de préférence 50 à 150, d'une charge minérale non siliceuse,
(E) : éventuellement 0,01 à 3 parties en poids d'un composé métallique catalytique de durcissement,
ladite dispersion ayant une teneur en extrait sec supérieure à 60 % en poids et une viscosité à 25°C inférieure à 50.000 mPa.s. mesurée à un gradient de vitesse de 1.s⁻¹ avant l'incorporation de la charge D.

2. Dispersion selon la revendication 1, caractérisée en ce que le latex B est filmogène ou non-filmogène dans les conditions ambiantes d'utilisation de ladite dispersion.

3. Dispersion aqueuse selon la revendication 1 ou 2, caractérisée en ce que l'émulsion (A) a une teneur en extrait sec d'au moins 40 % en poids et la dispersion (B) a une teneur en extrait sec comprise entre 20 et 70 % en poids.

4. Dispersion aqueuse selon l'une quelconque des revendications précédentes, caractérisée en ce qu'elle présente :
- une première population de particules provenant essentiellement de l'émulsion (A) ayant une granulométrie comprise entre 0,20 µm et 5 µm, et une deuxième population de particules provenant essentiellement de la dispersion (B) ayant une granulométrie comprise entre 0,01 µm et 0,10 µm,
- un extrait sec supérieur à 70 % en poids.

5. Dispersion aqueuse selon l'une quelconque des revendications précédentes, caractérisée en ce que la charge (D) est choisie parmi le carbonate de calcium, le noir de carbone, le dioxyde de titane, l'oxyde d'aluminium, l'alumine hydratée, la vermiculite expansée, le borax hydraté, la vermiculite non expansée, l'oxyde de zinc, le mica, le talc, l'oxyde de fer, le sulfate de baryum et la chaux éteinte et leurs divers mélanges possibles.

6. Dispersion aqueuse selon l'une quelconque des revendications précédentes, caractérisée en ce que la charge (D) est du carbonate de calcium de précipitation dont le diamètre particulaire moyen est inférieur à 0,1 µm.

7. Dispersion aqueuse selon l'une quelconque des revendications précédentes, caractérisée en ce que le composé métallique (E) est un sel d'organoétain se trouvant sous forme d'une émulsion aqueuse.

8. Dispersion aqueuse selon l'une quelconque des revendications précédentes, caractérisée en ce que l'on incorpore, en outre, pour 100 parties en poids d'émulsion A), 1 à 100 parties en poids d'une charge siliceuse renforçante ou semi-renforçante (F) choisie parmi les silices de pyrogénation, les silices de précipitation, les terres de diatomées, le quartz broyé et leurs mélanges.

9. Procédé de préparation d'une dispersion aqueuse telle que définie dans les revendications 1 à 8, caractérisé en ce qu'on introduit, dans l'ordre sous agitation à température ambiante, l'émulsion (A), la dispersion (B), l'agent de réticulation (C), le catalyseur (E) et la charge (D) et éventuellement l'additif silicié (F).

10. Utilisation d'une dispersion aqueuse telle que définie à l'une quelconque des revendications 1 à 8, comme peinture, comme revêtement de bouchons de liège et dans des compositions cosmétiques.

## Patentansprüche

1. Wäßrige Dispersion auf der Basis von Silicon, die durch Entfernung von Wasser unter Umgebungsbedingungen zu einem Elastomer aushärtet, dadurch gekennzeichnet, daß sie (in Gewicht) umfaßt:
(A) : 100 Teile einer Emulsion vom Typ Öl-in-Wasser eines α,ω-(Dihydroxy)-polydiorganosiloxans mit einer dynamischen Viskosität von mindestens 50.000 mPa·s bei 25 °C, wobei die Emulsion durch mindestens ein oberflächenaktives Mittel, ausgewählt unter den anionischen und den nichtionischen oberflächenaktiven Mitteln und ihren Mischungen, stabilisiert ist und eine Granulometrie zwischen 0,15 µm und 100 µm besitzt,
(B) : 2 bis 80 Teile, vorzugsweise 3 bis 40 Teile, einer wäßrigen Dispersion (Latex) eines organischen (Co)polymers, das eine Teilchengröße zwischen 0,01 und 0,15 µm und einen Trockenextrakt zwischen 20 und 70 Gew.-% aufweist, wobei das organische (Co)polymer unter den Copolymeren Styrol/Alkylacrylat, deren Gewichtsverhältnis Styrol/Acrylat zwischen 30/70 und 70/30 variiert, Polyvinylacetat-Homopolymer und Polystyrol-Homopolymer ausgewählt wird,
(C) : eine wirksame Menge von mindestens einem Vernetzungsmittel, ausgewählt unter:
. 1 bis 100 Teilen einer Mikroemulsion von Silsesquioxan-Harz,
. 5 bis 100 Teilen eines reaktiven Siliconharzes mit geringer Molmasse, das Alkoxy- und Acyloxygruppen aufweist,
. 5 bis 100 Teilen eines Siliconharzes mit hoher Molmasse, das in Toluol unlöslich ist,
. 5 bis 100 Teilen eines hydroxylierten Siliconharzes, das pro Molekül mindestens zwei unterschiedliche Einheiten aufweist, ausgewählt unter denen der Formeln: R₃SiO_{0,5} (M), R₂SiO (D), RSiO_{1,5} (T) und SiO₂ (Q), worin R hauptsächlich einen Alkylrest mit 1 bis 6 Kohlenstoffatomen, einen Vinylrest und einen 3,3,3-Trifluorpropylrest bedeutet und der Gehalt an Hydroxylgruppen zwischen 0,1 und 10 Gew.-% liegt,
(D) : 5 bis 200 Teile, vorzugsweise 50 bis 150 Teile, eines mineralischen, nicht silikathaltigen Füllstoffes,
(E) : gegebenenfalls 0,01 bis 3 Gewichtsteile einer katalytischen metallischen Härterverbindung,
wobei die genannte Dispersion vor dem Einbringen des Füllstoffes D einen Gehalt an Trockenextrakt von höher als 60 Gew.-% und eine Viskosität bei 25 °C von unter 50.000 mPa·s, gemessen bei einem Geschwindigkeits-Gradienten von 1.s⁻¹, besitzt.

2. Dispersion nach Anspruch 1, dadurch gekennzeichnet, daß Latex B unter den Umgebungsbedingungen zur Anwendung der genannten Dispersion filmbildend oder nicht filmbildend ist.

3. Wäßrige Dispersion nach Anspruch 1 oder 2, dadurch gekennzeichnet, daß die Emulsion (A) einen Gehalt an Trockenextrakt von mindestens 40 Gew.-% und die Dispersion (B) einen Gehalt an Trokkenextrakt zwischen 20 und 70 Gew.-% besitzen.

4. Wäßrige Dispersion nach irgendeinem der vorstehenden Ansprüche, dadurch gekennzeichnet, daß sie aufweist:
- eine erste Population von Teilchen, die im wesentlichen von der Emulsion (A) stammt und eine Granulometrie zwischen 0,20 µm und 5 µm besitzt, und eine zweite Population von Teilchen, die im wesentlichen von der Emulsion (B) stammt und eine Granulometrie zwischen 0,01 µm und 0,10 µm besitzt,
- einen Trockenextrakt von höher als 70 Gew.-%.

5. Wäßrige Dispersion nach irgendeinem der vorstehenden Ansprüche, dadurch gekennzeichnet, daß der Füllstoff (D) unter Calciumcarbonat, Aktivkohle, Titandioxid, Aluminiumoxid, hydratisierter Tonerde, geblähtem Vermiculit, hydratisiertem Borax, nicht geblähtem Vermiculit, Zinkoxid, Glimmer, Talk, Eisenoxid, Bariumsulfat, gelöschtem Kalk ihren verschiedenen möglichen Mischungen ausgewählt wird.

6. Wäßrige Dispersion nach irgendeinem der vorstehenden Ansprüche, dadurch gekennzeichnet, daß der Füllstoff (D) Calciumcarbonat aus der Fällung ist, dessen mittlerer Teilchendurchmesser unter 0,1 µm liegt.

7. Wäßrige Dispersion nach irgendeinem der vorstehenden Ansprüche, dadurch gekennzeichnet, daß die metallische Verbindung (E) ein Organozinnsalz ist, das in Form einer wäßrigen Emulsion vorliegt.

8. Wäßrige Dispersion nach irgendeinem der vorstehenden Ansprüche, dadurch gekennzeichnet, daß man außerdem pro 100 Gewichtsteile Emulsion (A) 1 bis 100 Gewichtsteile eines verstärkenden oder halbverstärkenden silikathaltigen Füllstoffes (F) einbringt, ausgewählt unter den Kieselerden aus der Verbrennung, den Kieselerden aus der Fällung, den Diatomeenerden, zerkleinertem Quarz und ihren Mischungen.

9. Verfahren zur Herstellung einer wäßrigen Dispersion wie in den Ansprüchen 1 bis 8 definiert, dadurch gekennzeichnet, daß man unter Rühren bei Umgebungstemperatur in der folgenden Reihenfolge einbringt: die Emulsion (A), die Dispersion (B), das Vernetzungsmittel (C), den Katalysator (E) und den Füllstoff (D) sowie gegebenenfalls den silikathaltigen Zusatz (F).

10. Verwendung einer wäßrigen Dispersion wie in irgendeinem der Ansprüche 1 bis 8 definiert, als Anstrichstoff, als Überzug für Korkstopfen und in kosmetischen Zusammensetzungen.

## Claims

1. Aqueous dispersion based on silicone crosslinking to an elastomer on removal of water in ambient conditions, characterized in that it comprises, by weight:
(A): 100 parts of an emulsion of the oil-in-water type of an α,ω-(dihydroxy)polydiorganosiloxane with a dynamic viscosity of at least 50 000 mPa s at 25°C, an emulsion stabilized with at least one surfactant selected from anionic and nonionic surface-active agents and their mixtures and having a particle size of between 0.15 and 100 µm,
(B): 2 to 80 parts, preferably 3 to 40, of an aqueous dispersion (latex) of an organic (co)polymer exhibiting a particle size of between 0.01 and 0.15 µm and a solids content of between 20 and 70 % by weight, the organic (co)polymer being selected from styrene/alkyl acrylate copolymers in which the styrene/acrylate weight ratio varies between 30/70 and 70/30, polyvinyl acetate homopolymer and polystyrene homopolymer.
(C): an effective quantity of at least one crosslinking agent selected from:
- from 1 to 100 parts of a silsesquioxane resin microemulsion,
- from 5 to 100 parts of a reactive silicone resin of low molecular mass, containing alkoxy and acyloxy groups,
- from 5 to 100 parts of a toluene-insoluble silicone resin of high molecular mass,
- from 5 to 100 parts of a hydroxylated silicone resin containing per molecule at least 2 different units selected from those of formulae: R₃SiO_{0,5} (M), R₂SiO (D), RSiO_{1.5} (T) and SiO₂ (Q), R being chiefly a C₁-C₆ alkyl, vinyl or 3,3,3-trifluoropropyl radical, and a weight content of hydroxyl group of between 0.1 and 10 %,
(D): 5 to 200 parts, preferably 50 to 150, of a non-siliceous inorganic filler,
(E): optionally 0.01 to 3 parts by weight of a cure catalyst metal compound,
the said dispersion having a solids content higher than 60 % by weight and a viscosity at 25°C lower than 50 000 mPa s, measured at a rate of gradient of 1 s⁻¹ before the incorporation of the filler D.

2. Dispersion according to claim 1, characterized in that the latex B is film-forming or not film-forming in the ambient conditions of use of the said dispersion.

3. Aqueous dispersion according to claim 1 or 2, characterized in that the emulsion (A) has a solids content of at least 40 % by weight and the dispersion (B) has a solids content of between 20 and 70 % by weight.

4. Aqueous dispersion according to any one of the preceding claims, characterized in that it exhibits:
- a first population of particles originating essentially from the emulsion (A), which has a particle size of between 0.20 µm and 5 µm, and a second population of particles originating essentially from the dispersion (B), which has a particle size of between 0.01 and 0.10 µm,
- a solids content higher than 70 % by weight.

5. Aqueous dispersion according to any one of the preceding claims, characterized in that the filler (D) is selected from calcium carbonate, carbon black, titanium dioxide, aluminium oxide, hydrated alumina, expanded vermiculite, hydrated borax, unexpanded vermiculite, zinc oxide, mica, talc, iron oxide, barium sulphate and slaked lime and their various possible mixtures.

6. Aqueous dispersion according to any one of the preceding claims, characterized in that the filler (D) is precipitated calcium carbonate whose mean particle diameter is smaller than 0.1 µm.

7. Aqueous dispersion according to any one of the preceding claims, characterized in that the metal compound (E) is an organotin salt which is in the form of an aqueous emulsion.

8. Aqueous dispersion according to any one of the preceding claims, characterized in that 1 to 100 parts by weight of a reinforcing or semireinforcing siliceous filler (F) are additionally incorporated, per 100 parts by weight of emulsion A), selected from pyrogenic silicas, precipitated silicas, diatomaceous earths, ground quartz and mixtures thereof.

9. Process for the preparation of an aqueous dispersion as defined in claims 1 to 8, characterized by the introduction, in this order, with stirring at ambient temperature, of the emulsion (A), the dispersion (B), the crosslinking agent (C), the catalyst (E) and the filler (D) and optionally the siliceous additive (F).

10. Use of an aqueous dispersion as defined in any one of claims 1 to 8, as paint, as coating for cork stoppers and in cosmetic compositions.
